# EUROPEAN PATENT APPLICATION

(11) **EP 4 197 458 A1**
(43) Date of publication of application: **21.06.2023**
(21) Application number: 21860185.4
(22) Date of filing: 13.08.2021
(51) Int. Cl.: A61B 17/12, A61B 17/00

(54) **HEMANGIOMA OCCLUSION APPARATUS, HEMANGIOMA OCCLUSION AND TREATMENT APPARATUS, AND HEMANGIOMA OCCLUSION SYSTEM**

(30) Priority: 31.08.2020 CN 202010899173
(71) Applicant: MicroPort NeuroTech (Shanghai) Co., Ltd., Shanghai 201318 (CN)
(72) Inventor: GUO, Shuang, Shanghai 201318 (CN); CHANG, Mengqi, Shanghai 201318 (CN); CHEN, Bing, Shanghai 201318 (CN); GUO, Yuanyi, Shanghai 201318 (CN); LU, Huina, Shanghai 201318 (CN); WANG, Yiqun Bruce, Shanghai 201318 (CN)
(74) Representative: Marks & Clerk LLP
(86) International application number: PCT/CN2021/112629
(87) International publication number: WO 2022/042347

(57) **Abstract**

An aneurysm occlusion device (10), a therapeutic apparatus for aneurysm occlusion and an aneurysm occlusion system are disclosed. The aneurysm occlusion device (10) includes at least an expandable mesh structure (11) having an expanded configuration with a planar spiral shape and a compressed configuration for delivery into an aneurysm (30) from a blood vessel. The expandable mesh structure (11) can be compressed within a catheter (40) and can recover the expanded configuration with the spiral shape after being released from the catheter (40). The aneurysm occlusion device (10) achieves stable, compliant packing while providing the advantages of among others, avoiding rupture of the aneurysm (30), preventing thrombosis in a blood vessel, increasing coverage of an opening at the aneurysm neck, promoting thrombosis in the aneurysm and accelerating embolization of the aneurysm (30).

## Description

### TECHNICAL FIELD

The present invention relates to the field of medical devices and, in particular, to an aneurysm occlusion device, a therapeutic apparatus for aneurysm occlusion and an aneurysm occlusion system.

### BACKGROUND

Intracranial aneurysms are pathological bulges in intracranial artery walls, with an incidence rate of 5% to 10%. A magnetic resonance angiography (MRA) study shows that approximately 7.0% of Chinese adults aged 35-75 years have unruptured aneurysms. Although subarachnoid hemorrhages caused by intracranial aneurysm ruptures account for about 5% of strokes, primary ruptures are associated with a mortality rate of 20-30%, and re-ruptures are associated with a mortality rate of as high as 60%. Aneurysm treatment relies on complete isolation of an aneurysm from blood circulation by a therapeutic method. Currently available therapeutic methods primarily include craniotomy and clipping and endovascular intervention. At present, endovascular intervention has been predominant because it allows direct access to a lesion without affecting the brain tissue and because of its minimally invasive nature. Currently available endovascular interventional therapies are principally as follows:
(1) At present, aneurysm embolization with coils is mostly used in aneurysm treatment. It can achieve aneurysm occlusion and treatment by promoting thrombosis through altering local hemodynamic factors. However, as aneurysms are diverse in shape and size, incomplete coil packing may lead to recanalization of an aneurysm, while excessive packing may cause intraoperative rupture of an aneurysm. This places stringent requirements on physicians' dexterity and experience. Moreover, coil packing may require multiple procedures and therefore lacks efficiency. In some cases, additional use of stents, balloons, and microcatheters may be required, leading to increased operational complexity. Further, for wide-neck aneurysms, coils tend to herniate into the parent arteries, affecting blood flow, or even causing vascular stenosis in severe cases.
(2) As a significant breakthrough in the field of endovascular treatment of intracranial aneurysms, flow diverters provide a novel approach for the treatment of complex aneurysms. This approach involves placing a dense mesh stent in a parent artery, which enables endoluminal reconstruction of the diseased blood vessel and subsequent luminal surface re-endothelialization by neointimal growth over the aneurysm neck. The use of flow diverters has significantly improved long-term therapeutic benefit for large and giant aneurysms and considerably reduced the use of coils. In addition, according to computer-aided hemodynamic simulation analysis, a metal coverage ratio of 30-50% can significantly reduce intra-aneurysmal blood flow and achieve a high cure rate. However, the use of flow diverters can lead to long-term reliance of patients on dual antiplatelet therapy and a risk of hemorrhagic complications after surgery. Further, some large aneurysms are associated with a risk of rupture a certain period of time after the treatment.
(3) Currently, there are also novel devices capable of embolization in one pass, which are generally made of shape memory materials and shaped into balls, cylinders or discs. Such a device is designed to be delivered through a catheter to a target site, where it is pushed out of a sheath and then self-expand to recover its spherical shape, thereby closing the aneurysm. For example, a first embolization device is a spherical or cylindrical dense mesh device with anchors at its opposite ends. After full expansion of the device within an aneurysm, a proximal dense mesh thereof covers the neck of the aneurysm to therapeutically treat the aneurysm. A second embolization device is a three-dimensional mesh-like structure composed of a radiopaque wire and surrounding self-expanding shape memory alloy. This device can be released and retrieved by a catheter like a coil and packed within an aneurysm in a spherical form capable of disturbing blood flow. A third embolization device is a braided double-layer nickel-titanium alloy device. A fourth embolization device is a braided double-layer shape memory alloy device, which is in the form of a coiled disc when not stressed. However, when released into an aneurysm, it will be stressed by walls of the aneurysm and comprises a tulip-like shape, which allows it to be stabilized in a lower part of the aneurysm while covering the aneurysm neck to provide an effect of hemodynamic remodeling. In the design of the first embolization device, the proximal anchor makes the device symmetric and necessary to cover the aneurysm neck in a certain orientation. Therefore, it is used mainly for the treatment of bifurcated wide-neck aneurysms and is particularly suitable for aneurysms with a regular morphology. Moreover, in the design of the first embolization device, the distal anchor may impact an aneurysm wall and is likely to cause a rupture or bleeding of the aneurysm. In some cases, the proximal anchor of the first embolization device may be pushed by the aneurysm wall to herniate into the parent artery, affecting the endothelialization of the aneurysm neck. In addition, the first embolization device is generally in the form of a single ball or cylinder, which is insufficiently strong despite of a large contact area. This is unfavorable to long-term stability, and tends to lead to displacement, of the device within the aneurysm. The three-dimensional mesh-like structure of the second embolization device is composed of multiple meshed sheets, and comprises a sphere-like shape. Due to significant friction between different parts of the three-dimensional mesh-like structure and between the structure itself and aneurysm walls, it is difficult for the device to restore the intended shape and, and even if this is achieved, the device cannot maintain the shape stably for a sufficiently long period of time. Therefore, it cannot provide desirable packing quality. Moreover, since this device must be used in combination with a coil, its operation is complicated. The third embolization device operates in a similar manner as the first embolization device and therefore suffers from similar problems. The fourth embolization device also has a proximal anchor, which likewise tends to be pushed by an aneurysm wall to herniate into the parent artery and makes the device suitable for use in apex aneurysms. Moreover, as this device requires repeated relocation before it can satisfactorily stay stably within the aneurysm, it lacks efficiency.

### SUMMARY OF THE INVENTION

In order to overcome the above problems, it is an object of the present invention to provide an aneurysm occlusion device, a therapeutic apparatus for aneurysm occlusion and an aneurysm occlusion system, which are used for treatment of an aneurysm by occlusion thereof, capable of recovering a preformed shape, more stably maintaining the shape within the aneurysm and causing less damage to aneurysm walls, associated with a reduced risk of rupture of the aneurysm, and able to be compressed to a smaller size enabling delivery within a catheter having a small inner diameter to a wider variety of lesions or delivery within a narrower blood vessel.

The above object is attained by an aneurysm occlusion device provided in the present invention, which comprises at least an expandable mesh structure having an expanded configuration with a planar spiral shape and a compressed configuration for delivery into an aneurysm from a blood vessel.

Optionally, the aneurysm occlusion device may further comprise a guide structure at least partially disposed in a lumen of the expandable mesh structure, wherein when the expandable mesh structure is in the spiral shape, at least a part of the guide structure in the lumen of the expandable mesh structure is expanded into a spiral shape.

Optionally, the aneurysm occlusion device may further comprise a guide structure at least partially disposed at an outer side of a distal end of the expandable mesh structure, wherein at least a part of the guide structure at an outer side of the distal end of the expandable mesh structure has an expanded configuration with a spiral shape and a compressed configuration for delivery into an aneurysm from a blood vessel.

Optionally, the guide structure may be entirely disposed at the outer side of the distal end of the expandable mesh structure, wherein a proximal end of the guide structure is coupled to the distal end of the expandable mesh structure.

Optionally, the guide structure may be configured as a planar spiral by spiraling a linear member about an axis, wherein the guide structure spirals in a same direction as the expandable mesh structure.

Optionally, the guide structure and the expandable mesh structure may spiral in a same plane, wherein an axis of spiraling of the guide structure coincides with an axis of spiraling of the expandable mesh structure.

Optionally, the planar spiral of the guide structure in its expanded configuration may be located within a first spiral turn of the expandable mesh structure proximate a distal end thereof.

Optionally, the expandable mesh structure may have a cross-sectional area increasing and then decreasing from a proximal end to a distal end thereof.

Optionally, the expandable mesh structure may comprise a proximal section, a middle section and a distal section, which are sequentially connected along an axis, wherein:
the proximal section has a cross-sectional area gradually increasing from a proximal end to a distal end; and/or the distal section has a cross-sectional area gradually increasing from a distal end to a proximal end.

Optionally, the cross-sectional area of the expandable mesh structure may repeatedly increase and decrease from a proximal end to a distal end.

Optionally, a maximum outer diameter of the expandable mesh structure may be 115 -1/2 of an outer diameter of the largest spiral turn in the expandable mesh structure.

Optionally, the maximum outer diameter of the expandable mesh structure may be 1/3-1/2 of the outer diameter of the largest spiral turn in the expandable mesh structure.

Optionally, the maximum outer diameter of the expandable mesh structure may be 2.0-10 mm, and the outer diameter of the largest spiral turn in the expandable mesh structure may be 3.0-30 mm.

Optionally, the distal end of the expandable mesh structure may be secured to a distal radiopaque ring. Alternatively or additionally, the proximal end of the expandable mesh structure may be fixedly coupled to a proximal radiopaque ring.

Optionally, the expandable mesh structure may be braided from 48-144 filaments made of a shape memory material and having a diameter of from 0.0005 inches to 0.002 inches.

Optionally, the expandable mesh structure may be braided from radiopaque filaments, or from a mixture of radiopaque and non-radiopaque filaments.

Optionally, the expandable mesh structure may have at least one spiral turn.

Optionally, the expandable mesh structure may have 1-5 spiral turns.

Optionally, the expandable mesh structure may have 1-3 spiral turns.

Optionally, the guide structure may have at least 1/4 spiral turn.

Optionally, the guide structure may have 1/4-3 spiral turns.

Optionally, the guide structure may have 1/4-2 spiral turns.

Optionally, an outer diameter of the largest spiral turn in the guide structure may not exceed 1/2 an outer diameter of the largest spiral turn in the expandable mesh structure.

Optionally, the guide structure may be made of a material containing a radiopaque material.

Optionally, a bulge radially extending outward may be provided at a proximal end of the expandable mesh structure.

Optionally, the bulge may be a closed ring, which is circumferentially continuous or non-continuous. Alternatively, the bulge may be an unclosed ring.

The above object is also attained by a therapeutic apparatus for aneurysm occlusion provided in the present invention, which comprises the aneurysm occlusion as defined above and a push pod. The push pod is coupled to the proximal end of the expandable mesh structure in the aneurysm occlusion device.

Optionally, the push pod may extend in a direction tangential to a spiral contour of the spiral shape of the expandable mesh structure in the expanded configuration.

The above object is also attained by an aneurysm occlusion system provided in the present invention, which comprises the aneurysm occlusion device as defined above and a catheter. The expandable mesh structure is configured to be compressed within the catheter and to recover the expanded configuration with the spiral shape after being released from the catheter.

Optionally, the catheter may have an inner diameter of 0.017, 0.021 or 0.027 inches.

The above object is also attained by a method of treating an aneurysm provided in the present invention. The aneurysm communicates, at a neck thereof, with a blood vessel. The method includes:
placing the aneurysm occlusion device as defined above within the aneurysm so that the expandable mesh structure in the aneurysm occlusion device spirals into a final shape in a plane of the aneurysm that intersects a plane where the neck of the aneurysm lies.

Optionally, the method may further comprise:
the expandable mesh structure spirals into a planar spiral in the plane, and an outer surface of the expandable mesh structure spans over the neck of the aneurysm so that the proximal end of the expandable mesh structure is sandwiched between an aneurysm wall and the outer surface.

Optionally, the method may further comprise:
releasing the guide structure within the aneurysm so that the guide structure spirals into a predetermined shape in the aneurysm and thereby defining a packing plane for the aneurysm; and
pushing and advancing the expandable mesh structure to release it, and the expandable mesh structure may spiral in the packing plane defined by the guide structure until it comprises the aforementioned expanded configuration, and enable the proximal end of the expandable mesh structure being oriented in parallel to the aneurysm wall and thus will not herniate into the blood vessel.

The aneurysm occlusion device includes at least an expandable mesh structure, which has an expanded configuration with a planar spiral shape and a compressed configuration for delivery into an aneurysm from a blood vessel. Since the planar spiral structure will encounter reduced friction within the aneurysm, the expandable mesh structure can more easily recover and maintain a predetermined shape in the aneurysm, which is suitable for occlusion of the aneurysm neck. Moreover, the distal end of the planar spiral structure is wrapped within the device or is not oriented toward the aneurysm wall, while the proximal end of the planar spiral structure is confined by pressure between the outer surface of the planar spiral and the aneurysm wall so as to be parallel to (including tangential scenarios) the aneurysm wall and thus has no impact on the aneurysm wall, avoiding the risk of rupture of the aneurysm. In addition, the outer surface of the largest spiral turn in the planar spiral structure covers and closes the aneurysm neck, thereby achieving a high coverage and facilitating embolization of the aneurysm. Further, a proximal anchor of the expandable mesh structure can avoid from being located at the middle of an opening at the aneurysm neck or from herniating. Furthermore, endothelialization over the aneurysm neck can be accelerated and the risk of vascular stenosis can be avoided.

In a loaded state of the expandable mesh structure for delivery from the blood vessel into the aneurysm, the planar spiral structure may be compressed into a linear shape and accommodated in a catheter. In this configuration, it has a radial size small enough to allow it to be received in the catheter that has a small inner diameter. In this way, it can be delivered to reach a wider variety of lesions or delivered within a narrower blood vessel, making it usable in an expanded spectrum of therapeutic applications. In particular, the planar spiral structure can be deployed without having to be oriented in a specific direction and thus can be suitably used to treat both regular and irregular aneurysms. Moreover, it can conform to different aneurysm shapes and can achieve aneurysm occlusion in a simpler manner. The device described herein can efficiently achieve embolization in one pass. Therefore, it allows simpler operation, less relies on experience of a physician implementing the surgical procedure and can reduce both surgical complexity and the required surgical time. Further, though covering the aneurysm neck opening with the outer surface of the largest spiral turn in the expandable mesh structure, since the outer diameter of the largest spiral turn matches an inner diameter of the aneurysm, a large contact area can be obtained, which imparts high supporting strength and enables stable occlusion by the aneurysm occlusion device without displacement.

The aneurysm occlusion device preferably further includes a guide structure. In one embodiment, the guide structure is at least partially disposed within a lumen of the expandable mesh structure. When the expandable mesh structure is in the spiral shape, the guide structure is at least partially expanded within the lumen of the expandable mesh structure into a spiral shape. With this arrangement, the inner guide structure can appropriately support the expandable mesh structure to improve the device's stability and reduce the risk of displacement. In another embodiment, the guide structure is at least partially disposed at an outer side of a distal end of the expandable mesh structure, and at least the part of the guide structure at the outer side of the distal end of the expandable mesh structure has an expanded configuration with a spiral shape. This design enables guidance of the expandable mesh structure by the spiral structure at the outer side of the distal end of the guide structure for more stable maintenance and easier recovery of the predetermined shape of expandable mesh structure, as well as for successful occlusion of the aneurysm neck. In particular, the guide structure may be entirely disposed at the outer side of the distal end of the expandable mesh structure and have an expanded configuration with a spiral shape. In this case, better guidance and even easier recovery of the predetermined shape of the expandable mesh structure in the aneurysm can be provided.

When the guide structure is entirely disposed at the outer side of the distal end of the expandable mesh structure, it is preferably configured as a planar spiral by spiraling a linear member around an axis, and the guide structure may spiral in the same direction as the expandable mesh structure. In this case, the guide structure may even better guide the expandable mesh structure for more stable maintenance and easier recovery of the predetermined shape of expandable mesh structure, as well as for successful occlusion of the aneurysm neck. Since the guide structure is elongate and flexible, less resistance to pushing will be encountered, and the entire device can be pushed and advanced more easily. Moreover, tension from the expandable mesh structure during its release can be relieved, reducing impact on the aneurysm wall and the risk of rupture of the aneurysm.

A cross-sectional area of the expandable mesh structure is preferred to increase and then decrease from the proximal end to the distal end, or repeatedly increase and decreased from the proximal end to the distal end. In this way, it is easier to configure the expandable mesh structure into a fusiform shape that is swollen at the middle and tapering to each end. This shape can facilitate compression of the device to a smaller size, increase flexibility of the device, additionally reduce resistance to pushing and reduce impact on the aneurysm wall. The distal end of the expandable mesh structure is preferred to be fixedly coupled to a distal radiopaque ring. This can, in particular when the guide structure is not included, increase smoothness at the distal end of the expandable mesh structure and thus additionally reduce possible damage caused to the aneurysm wall.

The expandable mesh structure may be braided from 48-144 filaments having a diameter of from 0.0008 inches to 0.002 inches. In this way, dense mesh openings can be formed, which allow easier embolization of the aneurysm. Moreover, the aneurysm wall can be more uniformly stressed, and the risk of rupture of the aneurysm can be additionally lowered.

A bulge radially extending outward may be provided at the proximal end of the expandable mesh structure. Through providing the bulge, friction between the expandable mesh structure and the aneurysm wall can be reduced, allowing the aneurysm occlusion device to be kept more stable and more resistant to displacement in the aneurysm. Moreover, the risk of the proximal end of the expandable mesh structure herniating into the parent blood vessel can be reduced, thereby additionally increasing coverage of the aneurysm neck, facilitating endothelialization over the aneurysm neck and accelerating embolization of the aneurysm.

The proximal end of the expandable mesh structure may be coupled to a push pod, and the push pod may extend in a direction tangential to a spiral contour of the spiral shape of the expandable mesh structure in the expanded configuration. This design facilitates coverage of the aneurysm neck opening by the outer surface of the largest spiral turn in the expandable mesh structure with the aid of the push pod, allowing even easier operation.

### BRIEF DESCRIPTION OF THE DRAWINGS

Fig. 1 is a diagram showing the structure of an aneurysm occlusion device according to a preferred embodiment of the present invention, in which each of a guide structure and an expandable mesh structure when in their expanded configurations have one spiral turn.
Fig. 2 is a diagram showing the structure of an aneurysm occlusion device according to a preferred embodiment of the present invention, in which a guide structure when in its expanded configuration has 1/2 spiral turn, and an expandable mesh structure when in its expanded configuration has 5/2 spiral turns.
Fig. 3 is a diagram showing the structure of an aneurysm occlusion device according to a preferred embodiment of the present invention, in which a guide structure is not included, and an expandable mesh structure when in its expanded configuration has 2 spiral turns.
Fig. 4 is a diagram showing the structure of an aneurysm occlusion device according to a preferred embodiment of the present invention, in which a guide structure is not included, a bulge is provided at a proximal end of an expandable mesh structure, and an expandable mesh structure when in its expanded configuration has 2 spiral turns.
Fig. 5 is a diagram showing an aneurysm occlusion device according to a preferred embodiment of the present invention in a configuration in which it is not completely released yet in an aneurysm.
Fig. 6 is a diagram showing an aneurysm occlusion device according to a preferred embodiment of the present invention in a configuration in which it is has been completely released in an aneurysm.
Fig. 7 is a partially enlarged view of an aneurysm occlusion device according to a preferred embodiment of the present invention, showing the coverage of an opening at the neck of an aneurysm.

### In these figures,

10, an aneurysm occlusion device;
11, an expandable mesh structure; 111, a distal end of an expandable mesh structure; 112, a proximal end of an expandable mesh structure; 113, a distal section; 114, a middle section; 115, a proximal section; 116, a bulge; 12, a guide structure; 121, a proximal end of a guide structure; 122, a distal end of a guide structure; 13, a distal radiopaque ring; 14, a proximal radiopaque ring; 20, a push pod; 30, an aneurysm; 40, a catheter; D1, an outer diameter of a largest spiral turn in a guide structure; D2, an outer diameter of a largest spiral turn in an expandable mesh structure; and D3, a maximum outer diameter of an expandable mesh structure.

Throughout the figures, the same reference numbers are used to denote the same or like elements.

### DETAILED DESCRIPTION

Objects, advantages and features of the present invention will become more apparent from the following detailed description when taken in conjunction with the accompanying drawings. Note that the figures are provided in a very simplified form not necessarily drawn to exact scale for the only purpose of facilitating easy and clear description of the disclosed embodiments.

As used herein, the singular forms "a", "an" and "the" include plural referents, unless the context clearly dictates otherwise. As used herein and in the appended claims, the term "or" is generally employed in the sense including "and/or", unless the context clearly dictates otherwise. The term "multiple" is generally employed in the sense including "two or more", unless the context clearly dictates otherwise. The term "several" is generally employed in the sense including "an indefinite number of', unless the context clearly dictates otherwise. The term "proximal end" generally refers to an end closer to an operator operating a medical device, and the term "distal end" generally refers to an end of the device that enters a human body first, unless the context clearly dictates otherwise.

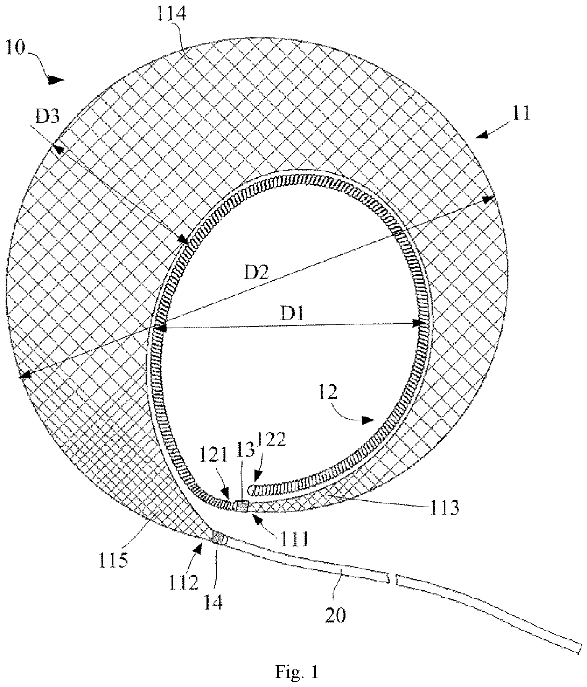

Reference is now made to Fig. 1, a diagram showing an aneurysm occlusion device 10 for treatment of an aneurysm by occlusion thereof according to an embodiment of the present invention. Examples of the aneurysm include, but are not limited to, intracranial aneurysms. Specifically, the aneurysm occlusion device 10 includes an expandable mesh structure 11 and a guide structure 12. The expandable mesh structure 11 has an expanded configuration with the shape of a planar spiral and a compressed configuration for delivery into the aneurysm from a blood vessel. It is preferred, but not mandatory, to include the guide structure 12.

In some embodiments, the guide structure 12 is at least partially disposed within a lumen of the expandable mesh structure 11. When the expandable mesh structure 11 is in the spiral shape, the guide structure 12 is at least partially expanded within the lumen of the expandable mesh structure 11 into a spiral shape. In this process, the part of the guide structure 12 within the expandable mesh structure 11 may spiral synchronously or asynchronously with the expandable mesh structure 11, but the present invention is not so limited. However, supporting the expandable mesh structure 11 with the guide structure 12 therein increases supporting strength of the entire device, ensures its stability and makes it less prone to displacement.

In some other embodiments, at least a part of the guide structure 12 is disposed at the outer side of a distal end of the expandable mesh structure 11 and has an expanded configuration with a spiral shape and a compressed configuration for delivery into the aneurysm from a blood vessel. Preferably, the guide structure 12 is entirely disposed at the outer side of the distal end of the expandable mesh structure 11. In this case, the guide structure 12 at outer side of the distal end of the expandable mesh structure 11, as a whole, has an expanded configuration with a spiral shape and a compressed configuration for delivery into the aneurysm from a blood vessel. Moreover, a proximal end 121 of the guide structure 12 is coupled to the distal end 111 of the expandable mesh structure 11, facilitating guidance of the expandable mesh structure 11 by the spiral of the guide structure located at outer side of the distal end. This enables the expandable mesh structure 11 to more easily recover a predetermined shape and more stably maintain the shape.

Further, the part of the guide structure 12 located at outer side of the distal end of the expandable mesh structure 11 is configured as a planar spiral or a three-dimensional spiral by spiraling a linear member about an axis. In the case of the part of the guide structure 12 being located at outer side of the distal end of the expandable mesh structure 11 being configured as a planar spiral, the guide structure 12 spirals in the same direction as the expandable mesh structure 11. Preferably, an axis of spiraling of the guide structure 12 coincides with an axis of spiraling of the expandable mesh structure 11, and the guide structure 12 and the expandable mesh structure 11 spiral in the same plane. In this way, guided by the guide structure 12 in the shape of a planar spiral located at outer side of the distal end, the expandable mesh structure 11 can more easily recover the planar spiral shape after being released into the aneurysm and more stably maintain the shape. Thus, the aneurysm can be effectively isolated, ensuring a desirable embolization effect. Alternatively, in the case of the part of the guide structure 12 being located at outer side of the distal end of the expandable mesh structure 11 being configured as a three-dimensional spiral, the guide structure 12 and the expandable mesh structure llmay spiral in the same direction or different directions. Preferably, the two spiral in the same direction in order to avoid bending of the spirals at the distal end 122 during delivery, which may affect shape recovery of the expandable mesh structure 11 within the aneurysm. In the case of the part of the guide structure 12 being located at outer side of the distal end of the expandable mesh structure 11 being configured as a three-dimensional spiral, an axis of spiraling of the guide structure 12 at the distal end 122 may coincide with an axis of spiraling of the expandable mesh structure 11, or not. However, the present invention is not limited in any way in this regard. Further, in the expanded configuration, the planar spiral structure of the part of the guide structure 12 located at outer side of the distal end of the expandable mesh structure 11 is situated within the first spiral turn proximate the distal end of the expandable mesh structure 11, for example, as shown in Fig. 1.

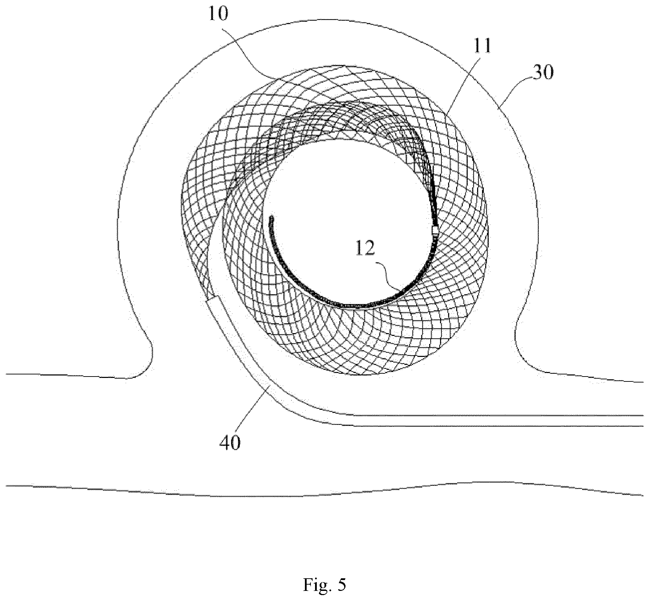

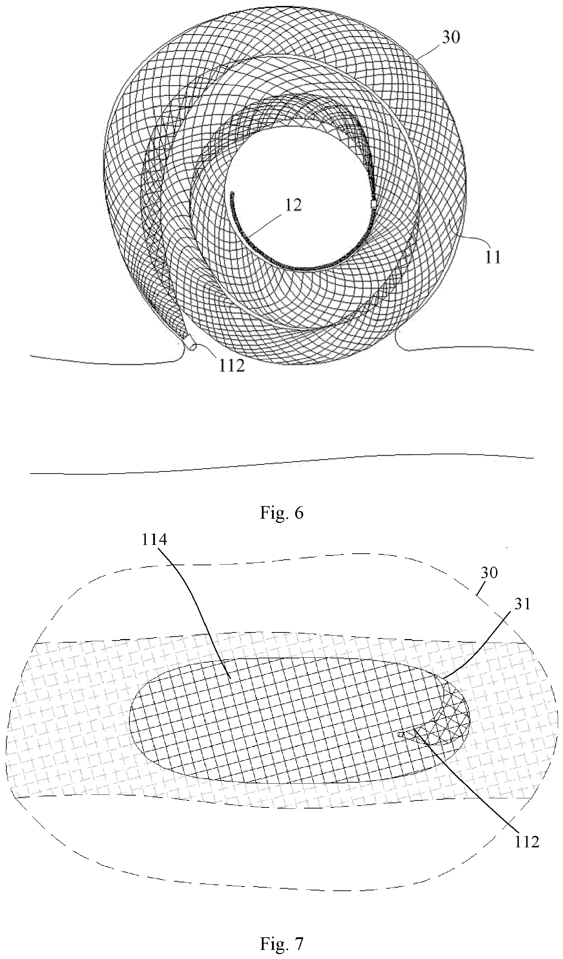

As noted above, the expandable mesh structure 11 has a compressed configuration and an expanded configuration. When in the expanded configuration, the expandable mesh structure 11 comprises the shape of a planar spiral. The compressed configuration of the expandable mesh structure 11 can facilitate delivery of the expandable mesh structure 11 through a catheter 40 into the aneurysm from a blood vessel. More specifically, the expandable mesh structure 11 is loaded in the catheter 40 (see Figs. 5 and 6) so as to be brought into the compressed configuration. In this configuration, the expandable mesh structure 11 generally comprises a linear shape and thus has a minimized radial size, which facilitates its delivery. When released from the catheter 40, the expandable mesh structure 11 can expand by its own elasticity until it comprises the expanded configuration. In this configuration, the expandable mesh structure 11 recovers the planar spiral shape. Likewise, the guide structure 12 also has a compressed configuration and an expanded configuration. In the expanded configuration, both the parts of the guide structure 12 within the expandable mesh structure 11 and at outer side of the distal end of the expandable mesh structure 11 recovers the spiral shape. The compressed configuration of the guide structure 12 can similarly facilitate delivery of the guide structure 12 through the catheter 40 into the aneurysm from a blood vessel. More specifically, the guide structure 12 is in the compressed configuration when it is loaded in the catheter 40. In this configuration, for example, the part(s) of the guide structure 12 at outer side of the distal end of the expandable mesh structure 11 and/or within the lumen of the expandable mesh structure 11 can each comprise a linear shape, which lead to a small radial size of the guide structure 12. Once released from the catheter 40, the guide structure 12 can expand by its own elasticity until it comprises the expanded configuration. In this configuration, for example, the part(s) of the guide structure 12 at outer side of the distal end of the expandable mesh structure 11 and/or within the lumen of the guide structure 12 recover(s) the spiral shape.

In embodiments of the present invention, there is also provided a therapeutic apparatus for aneurysm occlusion including the aneurysm occlusion device 10 and a push pod 20 for advancing the aneurysm occlusion device 10. The proximal end 112 of the expandable mesh structure 11 is configured to be detachably coupled to the push pod 20. Additionally, the push pod 20 is preferred to extend in a direction tangential to a spiral contour of the planar spiral of the expandable mesh structure 11 in the expanded configuration. In this way, the expandable mesh structure 11 can be advanced and released in a way that an outer surface of its largest spiral turn covers an opening 31 at the neck of an aneurysm (see Fig. 7). That is, the outer surface of the expandable mesh structure 11 spans across the neck of the aneurysm 30. This can enlarge the coverage of the aneurysm neck opening 31 and avoid herniation of the expandable mesh structure 11 at the proximal end 112. Moreover, it is ensured that the proximal end 112 of the expandable mesh structure 11 is not located at the middle of the aneurysm neck opening 31 and thus will not affect healing of the aneurysm neck. Without limiting the prevent invention, the detachment of the push pod 20 from the expandable mesh structure 11 may be accomplished thermally, electrically, mechanically, hydrolytically or otherwise as is conventional. The push pod 20 acts to push the aneurysm occlusion device 10 out of the catheter 40, resulting in the release of the aneurysm occlusion device 10 into the aneurysm 30.

Further, the guide structure 12 is preferred to be an elongate structure (i.e., a linear member) in its expanded configuration, which has an outer diameter much smaller than an outer diameter of the expandable mesh structure 11 when the latter is in the expanded configuration. In this way, compared with the expandable mesh structure 11, the guide structure 12 is more elongate and more flexible, thus providing a better guide and enabling the expandable mesh structure 11 to more easily recover the planar spiral shape in the aneurysm. Furthermore, when the guide structure 12 is located at outer side of the distal end of the expandable mesh structure 11, it can also function to relieve tension from the expandable mesh structure 11 during its release, thereby reducing impact on the wall of the aneurysm and resistance to pushing and making the pushing easier.

At least a part of the guide structure 12, especially the portion within the expandable mesh structure 11, is preferred to be radiopaque. To this end, the guide structure 12 may be wholly or partially made of a radiopaque material. The radiopaque material may be selected, for example, as platinum (Pt), a platinum-iridium (Pt-Ir) alloy, gold (Au) or the like, without limiting the present invention. In one embodiment, the linear member of the guide structure 12 may be formed by densely winding a metal wire (e.g., a platinum-tungsten alloy, a nickel-titanium alloy, stainless steel or the like) on a metal core bar into a primary coil, and the spiral-shaped guide structure 12 may be obtained by subjecting the primary coil (i.e., linear member) to a shaping process using a mold with a predetermined shape. In another embodiment, the linear member of the guide structure 12 may be a flexible mesh tube formed by cutting a metal tube, and the spiral-shaped guide structure 12 may be obtained by elongating the flexible meshtube and then subjecting it to a shaping process. In yet another embodiment, the linear member of the guide structure 12 may be a braided tube, and the spiral-shaped guide structure 12 may be obtained by elongating the braided tube and then subjecting it to a shaping process. In other embodiments, an elongate shape can be obtained without an elongation process.

The expandable mesh structure 11 is preferably formed by spirally winding braided tube. Preferably, the braided tube is formed of 48-144 filaments with a diameter of 0.0005-0.002 inches. In this way, a dense mesh with a high mesh opening density can be constructed, which can effectively block blood flow and promote thrombosis in the aneurysm and allows improved coverage of the aneurysm neck. Materials suitable for fabrication of the filaments may include shape memory materials, which may be metal materials with shape memory properties, such as nickel titanium (Ni-Ti) alloys, nickel-titanium-cobalt alloys (Ni-Ti-Co), double-layer composite metal wires (e.g., Ni-Ti@Pt), etc. The material of the filaments may also be a polymer material with certain shape recovery properties, such as polydioxanone (PDO), poly(l-lactide-co-ε-caprolactone) (PLC), polyurethane (PU), amorphous polynorbornene or a combination thereof. Herein, the filaments are made of a shape memory metal or a polymer material with certain shape recovery properties, which imparts shape memory and recovery properties to the mesh. Preferably, the expandable mesh structure 11 is braided from radiopaque filament. Alternatively, the expandable mesh structure 11 may be braided from both radiopaque and non-radiopaque filaments. With this design, the expandable mesh structure 11 will be radiopaque to X-ray fluoroscopy, while exhibiting sufficient elasticity, which enables the expandable mesh structure 11 to have strong ability to recover and maintain its original shape. The present invention is not limited to any particular radiopaque material of the radiopaque filaments, and for example, platinum (Pt), platinum iridium alloys (Pt-Ir), gold (Au) and the like can be suitably used.

In the expanded configuration, the guide structure 12 is preferred to have no more than 3 spiral turns and has at least 1/4 spiral turn. More preferably, it has 1/4 to 2 spiral turns. Considering that an excessive outer diameter of the spiral of the guide structure 12 tends to adversely affect the advancement or expansion of the expandable mesh structure 11, an outer diameter D1 of the largest spiral turn of the guide structure 12 is limited to a value not exceeding 1/2 of the outer diameter D2 of the largest spiral turn in the expandable mesh structure 11. When the number of spiral turns exceeds 5, the aneurysm occlusion device 10 will experience increased resistance to advancement and greater friction during shape recovery in the aneurysm. As a result, the expandable mesh structure 11 is less likely to recover its planar spiral shape. Therefore, in the expanded configuration, the expandable mesh structure 11 is preferred to have no more than 5 spiral turns and may have at least 1, more preferably, 1-3 spiral turns.

In an exemplary embodiment, as shown in Fig. 1, the guide structure 12 has 1 spiral turn when in the expanded configuration, and the expandable mesh structure 11 also has 1 spiral turn when in its expanded configuration. In this case, the proximal end 112 and the distal end 111 of the expandable mesh structure 11 are preferably located at the same radial location of the expandable mesh structure 11 on the same side of the axis of spiraling. More preferably, the outer diameter D1 of the largest spiral turn of the guide structure 12 is 1/2 of the outer diameter D2 of the largest spiral turn of the expandable mesh structure 11. Additionally, a maximum outer diameter D3 of (the lumen of) the expandable mesh structure 11 is preferred to be 1/2 of the outer diameter D2 of its largest spiral turn. It would be appreciated that, in the case of the planar spiral shape, the outer diameter of the largest spiral turn is just the maximum outer diameter of the spiral, while in the case of the three-dimensional spiral shape, the outer diameter of the largest spiral turn refers to a maximum outer diameter of a projection of the spiral on a plane perpendicular to its axial direction.

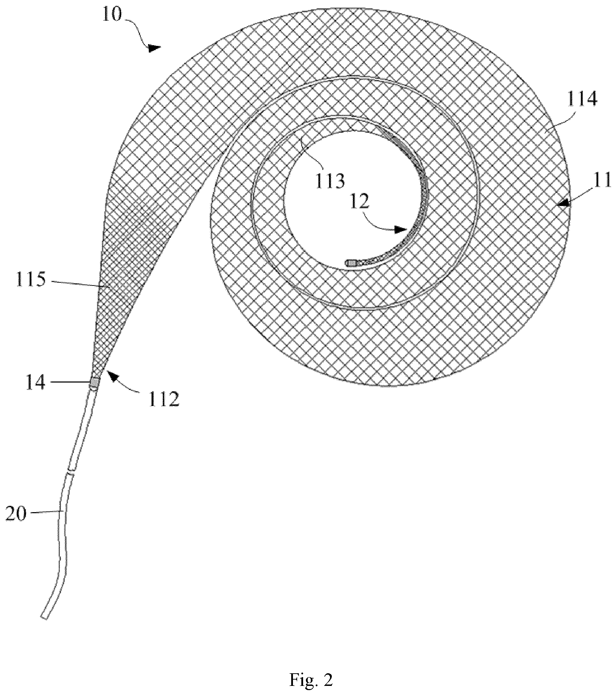

In another exemplary embodiment, as shown in Fig. 2, the guide structure 12 has 1/2 spiral turn when in its expanded configuration, while the expandable mesh structure 11 has 5/2 spiral turns when in the expanded configuration. In this case, the outer diameter D1 of the largest spiral turn in the guide structure 12 is preferred to be 1/3 of the outer diameter D2 of the largest spiral turn in the expandable mesh structure 11. Moreover, the maximum outer diameter D3 of the expandable mesh structure 11 is preferred to be 1/3 of the outer diameter D2 of the largest spiral turn. The outer diameter D2 of the largest spiral turn in the expandable mesh structure 11 may be configured according to the actual size of the aneurysm. Optionally, the maximum outer diameter D3 of the expandable mesh structure 11 may lie between 2 mm and 10 mm and the outer diameter D2 of the largest spiral turn between 3 mm and 30 mm.

Additionally, the expandable mesh structure 11 preferably has a cross-sectional area increasing and then decreasing from the proximal end 112 to the distal end 111. That is, the outer diameter of the expandable mesh structure 11 is not constant. Further, the expandable mesh structure 11 includes a distal section 113, a middle section 114 and a proximal section 115 that are sequentially connected along an axis. The distal section 113 preferably has a cross-sectional area (or diameter) increasing from the distal end 111 to the middle section 114, and/or the proximal section 115 preferably has a cross-sectional area (or diameter) increasing from the proximal end 112 to the middle section 114. In other embodiments, the cross-sectional area of the expandable mesh structure 11 may repeatedly increase and decrease from the proximal end 112 to the distal end 111. That is, it may repeatedly widen and narrow. This design can facilitate configuration of the expandable mesh structure 11 into a fusiform shape (swollen at the middle and tapering to each end), which in turn facilitates compression thereof into a smaller size. Moreover, it enables the aneurysm occlusion device to be more flexible, and pushed and advanced while experiencing less resistance and exerting a reduced impact on the aneurysm wall. The distal section 113 can provide a guiding function, which facilitates shaping of the expandable mesh structure 11. This design enables the expandable mesh structure 11 to have a greater mesh opening density at the proximal section 115 and an even greater mesh opening density at the distal section 113. It also imparts to the aneurysm occlusion device 10 increased strength, better support and stability within the spiral and improved resistance to displacement. The present is not limited to any particular mesh opening density distribution of the middle section 114, and for example, the middle section 114 may have a uniform or non-uniform mesh opening density.

When in the expanded configuration, the maximum outer diameter D3 of the expandable mesh structure 11 is not smaller than 115 of the outer diameter D2 of the largest spiral turn in the expandable mesh structure 11 and is preferably equal to 1/5-1/2, more preferably 1/3-1/2 thereof. One advantage of this design is that sufficient friction is ensured between the outer surface of the largest spiral turn in the expandable mesh structure 11 and the aneurysm wall to decrease the likeliness of displacement of the aneurysm occlusion device 10. Another advantage is that support force of the aneurysm occlusion device 10 is dispersed, avoiding damage to aneurysm wall possibly caused by local excessive pressure. A third advantage is that it is ensured that the outer surface of the largest spiral turn in the expandable mesh structure 11 can cover the aneurysm neck as completely as possible, so as to increase coverage of the aneurysm neck and hence to accelerate thrombosis within the aneurysm.

Further, in order to facilitate size control of the spiral, during actual fabrication, adjacent spiral turns in the planar spiral guide structure 12 are preferably closely brought together. For example, in the case of multiple spiral turns, an outer wall of the first spiral turn is closely fitted against an inner wall of the second spiral turn, an outer wall of the second spiral turn is closely fitted against an inner wall of the third spiral turn, and so forth. Likewise, adjacent spiral turns in the expandable mesh structure 11 are preferably closely brought together. Moreover, an inner wall of the first spiral turn of the expandable mesh structure 11 proximate the distal end (i.e., an inner wall of the expandable mesh structure 11 itself) is preferably closely fitted against an outer wall of the outermost spiral turn in the guide structure 12 proximate the proximal end (i.e., the outer surface of the guide structure 12 itself) to facilitate shaping of the expandable mesh structure 11 under the guidance of the guide structure 12. Further, when the guide structure 12 is in the shape of a three-dimensional spiral, preferably, the outer diameter of the largest spiral turn thereof does not exceed an outer diameter of the first spiral turn of the expandable mesh structure 11 proximate the distal end. For example, in the case of the guide structure 12 having multiple spiral turns, the outer diameter of the first spiral turn of the guide structure 12 proximate the distal end is preferably smaller than an outer diameter of each remaining spiral turn, and an outer diameter of each remaining spiral turn of the guide structure 12 does not exceed the outer diameter of the first spiral turn of the expandable mesh structure 11 proximate distal end. Each remaining spiral turn of the guide structure 12 may have an equal or unequal outer diameter and preferably has an equal outer diameter. In addition, the outer diameter of the first spiral turn of the guide structure 12 proximate distal end may be not smaller than 2/3 of the outer diameter of each remaining spiral turn of the guide structure 12, and the outer diameter of each remaining spiral turn of the guide structure 12 is more preferably not smaller than 2/3 of the outer diameter of the first spiral turn in the expandable mesh structure 11 proximate distal end. This design is advantageous in, during release and shape recovery of the expandable mesh structure 11, providing a space for planar packing of the expandable mesh structure 11 and avoiding the spiral of the guide structure 12 from occupying additional space to affect the release and shape recovery of the expandable mesh structure 11 in the aneurysm. It would be appreciated that the first spiral turn of the guide structure 12 refers to the spiral turn at the most distal end thereof and that the first spiral turn of the expandable mesh structure 11 likewise refers to the spiral turn at the most distal end thereof.

The aneurysm occlusion device 10 may further include a distal radiopaque ring 13 (see Fig. 1) disposed at the distal end 111 of the expandable mesh structure 11 or at the distal end 122 of the guide structure 12. By virtue of the radiopaqueness of the distal radiopaque ring 13 to X-ray fluoroscopy, the position of the distal end of the expandable mesh structure 11 or of the proximal end of the guide structure 12 can be determined. Preferably, the distal end 111 of the expandable mesh structure 11 is coupled by the distal radiopaque ring 13 to the guide structure 12, for example, to the proximal end 121 of the guide structure 12. Specifically, a distal end portion of the expandable mesh structure 11 may be gathered, and the distal radiopaque ring 13 may be disposed thereover. The two may be secured together by welding or gluing. Additionally, a proximal end portion of the guide structure 12 may be likewise received in the distal radiopaque ring 13 and secured thereto by welding or gluing. In other embodiments, the distal end 111 of the expandable mesh structure 11 may be directly coupled to the proximal end 121 of the guide structure 12, rather than by the distal radiopaque ring 13. For example, the distal end of the expandable mesh structure 11 may be coupled by an anti-unwinding wire, and secured by a light-curable adhesive, to the proximal end of the guide structure 12. This is advantageous in that the distal radiopaque ring 13 can be omitted, increasing flexibility of the expandable mesh structure 11 at the distal end 111 and thereby reducing resistance to advancement. In one embodiment, the guide structure 12 may consist of a helically wound metal coil, which may have an anti-unwinding wire portion. In this case, the anti-unwinding wire portion of the metal coil may be simply coupled and fixed to filaments in the expandable mesh structure 11 at the distal end thereof.

The aneurysm occlusion device 10 may further include a proximal radiopaque ring 14 (see Fig. 1) disposed at the proximal end 112 of the expandable mesh structure 11. By virtue of the radiopaqueness of the proximal radiopaque ring 14 to X-ray fluoroscopy, the position of the proximal end of the expandable mesh structure 11 can be determined. At the proximal end 112 of the expandable mesh structure 11, a connecting member (not shown) configured to be detachably coupled to the push pod 20 may be additionally provided. Additionally, in order to reduce possible damage caused to the aneurysm wall, the guide structure 12 may be provided at the distal end 122 with a smooth hemispherical structure formed by a light-curable adhesive. It would be appreciated that, when the expandable mesh structure 11 is braided from radiopaque filaments itself, the proximal radiopaque ring 14 and the distal radiopaque ring 13 may be either included or not. It would be also appreciated that when the guide structure 12 is included, the distal radiopaque ring may be provided either at the distal end 122 of the guide structure 12, or at the proximal end 121 of the guide structure 12 (i.e., at the distal end 111 of the expandable mesh structure 11). Alternatively, only the distal radiopaque ring may be provided at the distal end 122 of the guide structure 12 or at the proximal end 121 of the guide structure 12.

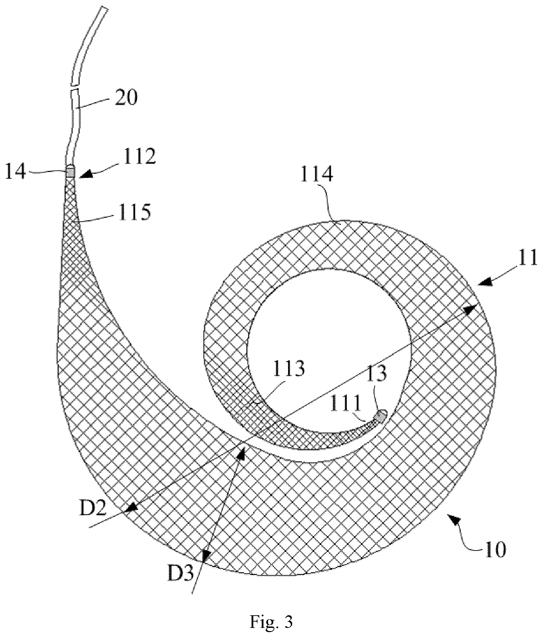

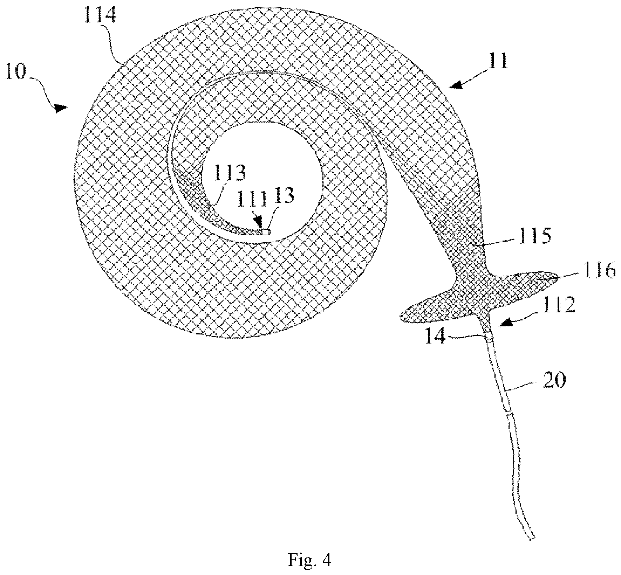

In alternative embodiments, the guide structure 12 may be omitted from the aneurysm occlusion device 10, for example, as shown in Figs. 3 and 4. In these cases, the occlusion of the aneurysm 30 can be accomplished only by the expandable mesh structure 11, and stable and compliant embolization can be likewise achieved. Moreover, in order to make the distal end 111 of the expandable mesh structure 11 smooth enough not to cause damage to the aneurysm wall, the distal end 111 is preferably harnessed and secured by the distal radiopaque ring 13 and/or bonded by an adhesive. Additionally, when the guide structure 12 is omitted, the expandable mesh structure 11 may have the aforementioned distal section 113, which replaces the guide structure 12 to provide a guiding function. Further, when the guide structure 12 is omitted, the expandable mesh structure 11 may have the aforementioned proximal section 115, which can facilitate compression and fitting onto the outer wall of the largest spiral turn. In this way, impact on the aneurysm wall can be reduced, and the aneurysm occlusion device 10 can be kept stable within the aneurysm. Furthermore, when the guide structure 12 is omitted, as shown in Figs. 3 and 4, the expandable mesh structure 11 may have multiple spiral turns, such as 2, 2.5 or more spiral turns. Preferably, the number of spiral turns is not greater than 5 and not smaller than 1. More preferably, the maximum outer diameter D3 of the expandable mesh structure 11 is 1/2 of the outer diameter D2 of the largest spiral turn.

Further, in order to increase stability of the aneurysm occlusion device 10, a bulge radially extending outward 116 is preferably provided at the proximal end 112 of the expandable mesh structure 11, as shown in Fig. 4. It would be appreciated that, the bulge 116 may be provided at the proximal end 112 of the expandable mesh structure 11 when the guide structure 12 is included in the aneurysm occlusion device 10. Providing the bulge 116 can increase friction between the expandable mesh structure 11 and the aneurysm wall, enhance stability of the device, reduce the risk of herniation of the proximal section 115, lower the risk of thrombosis in a blood vessel, improve coverage of the aneurysm neck and accelerate embolization of the aneurysm. In some embodiments, the bulge 116 may be a circumferentially continuous closed flange (as shown in Fig. 4). That is, the bulge 116 is made up of one circumferentially continuous projection structure. In some other embodiments, the bulge 116 may be a circumferentially non-continuous closed flange. That is, the bulge 116 is made up of multiple projection structures that are spaced apart circumferentially. In yet some other embodiments, the bulge 116 is an unclosed ring extending circumferentially around the proximal section 115, such as for example, a 1/4 ring, a half ring or a 3/4 ring. The present invention is not limited to any particular shape of the bulge 116, and possible shapes may include, but are not limited to, ellipses, circles, etc.

How the aneurysm occlusion device 10 of the present invention operates will be described below with reference to Figs. 5 to 7, in which the guide structure 12 comprises the shape of a planar spiral, as an example.

At first, the aneurysm occlusion device 10 is loaded into the catheter 40 for delivery. The aneurysm occlusion device 10 is loaded in the compressed configuration, in which both the expandable mesh structure 11 and the guide structure 12 are elongated into linear shapes, resulting in a radial size of the device that is small enough to allow the device to be accommodated in the catheter 40 having a small inner diameter. Optionally, the inner diameter of the catheter 40 may be 0.017 inches, 0.021 inches or 0.027 inches. After that, as shown in Fig. 5, a distal end of the catheter 40 is held still over the neck of the aneurysm 30, and the aneurysm occlusion device 10 is released. In the release process, the push pod 20 (not shown in Figs. 5 and 6) may be manipulated to distally push the device 10 or proximally retract the catheter 40 to release the guide structure 12 into the aneurysm 30. Tthe guide structure 12 recoils into its predetermined spiral shape in the aneurysm. Since the guide structure 12 is an elongate flexible planar spiral structure, it encounters less friction in the aneurysm and thus can easily recover the planar spiral shape, which defines a packing plane with an orientation corresponding to a maximum extent of stretching of the guide structure 12 in the aneurysm. When the aneurysm occlusion device 10 is further pushed, release of the expandable mesh structure 11 will start. Under the guidance of the guide structure 12, the expandable mesh structure 11 increasingly spirals into the packing plane defined by the guide structure 12, while the outer surface of the guide structure 12 is fitting against an inner surface of the expandable mesh structure 11. The outer diameter of the spiral of the expandable mesh structure 11 expands until the expandable mesh structure 11 is completely expanded, obtaining the packing configuration as shown in Fig. 6. At this time, the outer surface of the largest spiral turn in the aneurysm occlusion device 10 covers the aneurysm neck from an inner side thereof, and the whole device stably spirals within the aneurysm 30, achieving stable and compliant packing. Finally, after full packing is confirmed, the push pod 20 may be electrically detached from the expandable mesh structure 11, and the catheter 40 and the push pod 20 are withdrawn, ending the process.

Referring to Fig. 6, a distal end of the aneurysm occlusion device 10 (i.e., the distal end of the guide structure 12 or the distal end of the expandable mesh structure 11) is wrapped within the planar spiral structure and has no impact on the aneurysm wall. Moreover, the proximal end 112 of the expandable mesh structure is confined between the outer surface of the largest spiral turn and an aneurysm wall and is oriented in parallel to the aneurysm wall. Therefore, it has little impact on the aneurysm wall. In addition, the portion contacting the aneurysm wall is a braided dense mesh. This enables the aneurysm wall to be more uniformly stressed and less damaged. In particular, the planar spiral structure may be composed of multiple spiral turns, which are arranged radially side by side. These spiral turns act as barriers within aneurysm, which can better block blood flow and facilitate thrombosis, thus accelerating embolization of the aneurysm. With additional reference to Fig. 7, it is the outer surface of the largest spiral turn in the middle section 114 of the expandable mesh structure 11 that closes the opening 31 at the aneurysm neck by fitting against the inner wall of the aneurysm 30. This enables the device to have good stability. Since the proximal end of the expandable mesh structure 12 is oriented to be parallel to the aneurysm wall, herniation into the parent blood vessel will unlikely occur and endothelialization over the aneurysm neck will not be affected. Moreover, healing of the opening at the aneurysm neck can be accelerated, and good embolization results can be obtained.

It would be appreciated that the aneurysm occlusion device 10 is so deployed in the aneurysm 30 that the expandable mesh structure 11 spirals in a plane of the aneurysm 30 that intersects a plane where the neck of the aneurysm 30 lies. The expandable mesh structure 11 spirals in said plane, and the outer surface of the expandable mesh structure 11 spans over the neck of the aneurysm 30 so that the proximal end 112 of the expandable mesh structure 11 is sandwiched between the aneurysm wall and the outer surface.

In embodiments of the present invention, there is also provided an aneurysm occlusion system including the aneurysm occlusion device 10 and the catheter 40. The expandable mesh structure 11 is compressed and contracted within the catheter 40 and recovers an expanded configuration with a spiral shape after being released from the catheter 40.

In summary, compared with the prior art, the therapeutic apparatus for aneurysm occlusion of the present invention allows release with a simpler operation, provides high embolization efficiency, less relies on experience of a physician implementing the surgical procedure and can reduce the required surgical time. Moreover, it can cover the neck of an aneurysm without having to be oriented in a specific direction and thus can be suitably used to treat both regular and irregular aneurysms. It comprises a stable shape within an aneurysm while ensuring effective occlusion of an opening at the aneurysm neck, and prevents its proximal end from being located at middle of the opening at aneurysm neck or from herniating. Further, the distal end is wrapped or oriented inwardly and will not have an impact on the aneurysm wall, avoiding the risk of rupture of the aneurysm.

The description presented above is merely that of some preferred embodiments of the present invention and does not limit the scope thereof in any sense. Any and all changes and modifications made by those of ordinary skill in the art based on the above teachings fall within the scope as defined in the appended claims.

## Claims

1. An aneurysm occlusion device, comprising at least an expandable mesh structure, wherein the expandable mesh structure has an expanded configuration with a planar spiral shape and a compressed configuration for delivery into an aneurysm from a blood vessel.

2. The aneurysm occlusion device of claim 1, further comprising a guide structure at least partially disposed in a lumen of the expandable mesh structure, wherein when the expandable mesh structure is in the spiral shape, at least a part of the guide structure in the lumen of the expandable mesh structure is expanded into a spiral shape.

3. The aneurysm occlusion device of claim 1, further comprising a guide structure at least partially disposed at an outer side of a distal end of the expandable mesh structure, wherein at least a part of the guide structure at the outer side of the distal end of the expandable mesh structure has an expanded configuration with a spiral shape and a compressed configuration for delivery into an aneurysm from a blood vessel.

4. The aneurysm occlusion device of claim 3, wherein the guide structure is entirely disposed at the outer side the distal end of the expandable mesh structure, and wherein a proximal end of the guide structure is coupled to the distal end of the expandable mesh structure.

5. The aneurysm occlusion device of claim 4, wherein the guide structure is configured as a planar spiral by spiraling a linear member about an axis, and wherein the guide structure spirals in a same direction as the expandable mesh structure.

6. The aneurysm occlusion device of claim 5, wherein the guide structure and the expandable mesh structure spiral in a same plane, and wherein an axis of spiraling of the guide structure coincides with an axis of spiraling of the expandable mesh structure.

7. The aneurysm occlusion device of claim 5, wherein the planar spiral of the guide structure in the expanded configuration is located within a first spiral turn of the expandable mesh structure proximate a distal end thereof.

8. The aneurysm occlusion device of any one of claims 1 to 7, wherein the expandable mesh structure has a cross-sectional area increasing and then decreasing from a proximal end to a distal end.

9. The aneurysm occlusion device of claim 8, wherein the expandable mesh structure comprises a proximal section, a middle section and a distal section which are sequentially connected along an axis, and wherein: the proximal section has a cross-sectional area gradually increasing from a proximal end to a distal end; and/or the distal section has a cross-sectional area gradually increasing from a distal end to a proximal end.

10. The aneurysm occlusion device of claim 8, wherein the cross-sectional area of the expandable mesh structure repeatedly increases and decreases from a proximal end to a distal end.

11. The aneurysm occlusion device of claim 8, wherein a maximum outer diameter of the expandable mesh structure is 115 -1/2 of an outer diameter of a largest spiral turn in the expandable mesh structure.

12. The aneurysm occlusion device of any one of claims 1 to 7, wherein: a distal end of the expandable mesh structure is secured to a distal radiopaque ring; and/or a proximal end of the expandable mesh structure is fixedly coupled to a proximal radiopaque ring.

13. The aneurysm occlusion device of any one of claims 1 to 7, wherein the expandable mesh structure is braided from 48-144 filaments made of a shape memory material and having a diameter of from 0.0005 inches to 0.002 inches.

14. The aneurysm occlusion device of claim 13, wherein: the expandable mesh structure is braided from radiopaque filaments; or from a mixture of radiopaque and non-radiopaque filaments.

15. The aneurysm occlusion device of any one of claims 1 to 7, wherein the expandable mesh structure has at least one spiral turn.

16. The aneurysm occlusion device of claim 15, wherein the expandable mesh structure has 1 to 5 spiral turns.

17. The aneurysm occlusion device of any one of claims 2 to 7, wherein the guide structure has at least 1/4 spiral turn.

18. The aneurysm occlusion device of claim 17, wherein the guide structure has 1/4-3 spiral turns.

19. The aneurysm occlusion device of any one of claims 2 to 7, wherein an outer diameter of a largest spiral turn in the guide structure does not exceed 1/2 of an outer diameter of a largest spiral turn in the expandable mesh structure.

20. The aneurysm occlusion device of any one of claims 2 to 7, wherein the guide structure is made of a material containing a radiopaque material.

21. The aneurysm occlusion device of any one of claims 1 to 7, wherein a bulge radially extending outward is provided at a proximal end of the expandable mesh structure.

22. The aneurysm occlusion device of claim 21, wherein: the bulge is a closed ring, which is circumferentially continuous or non-continuous; or the bulge is an unclosed ring.

23. A therapeutic apparatus for aneurysm occlusion, comprising the aneurysm occlusion device of any one of claims 1 to 22 and a push pod, wherein the push pod is coupled to a proximal end of the expandable mesh structure in the aneurysm occlusion device.

24. The therapeutic apparatus for aneurysm occlusion of claim 23, wherein the push pod extends in a direction tangential to a spiral contour of the spiral shape of the expandable mesh structure in the expanded configuration.

25. An aneurysm occlusion system, comprising the aneurysm occlusion device of any one of claims 1 to 22 and a catheter, wherein the expandable mesh structure is configured to be compressed within the catheter and to recover the expanded configuration with the spiral shape after being released from the catheter.

26. The aneurysm occlusion system of claim 25, wherein the catheter has an inner diameter of 0.017, 0.021 or 0.027 inches.
